(19) Europäisches Patentamt
European Patent Office
Office européen des brevets

(11) Veröffentlichungsnummer: **0 191 318**
**A2**

(12) # EUROPÄISCHE PATENTANMELDUNG

(21) Anmeldenummer: **86100724.3**

(22) Anmeldetag: **21.01.86**

(51) Int. Cl.$^4$: **G 03 G 5/06**, C 07 D 471/14

(30) Priorität: **26.01.85 DE 3502681**

(43) Veröffentlichungstag der Anmeldung: **20.08.86**
**Patentblatt 86/34**

(84) Benannte Vertragsstaaten: **DE FR GB NL**

(71) Anmelder: **HOECHST AKTIENGESELLSCHAFT,**
**Postfach 80 03 20, D-6230 Frankfurt am Main 80 (DE)**

(72) Erfinder: **Wiedemann, Wolfgang, Dr. Dipl.-Chem.,**
**Pulignystrasse 14, D-6225 Geisenheim-Johannisberg (DE)**
Erfinder: **Günther, Dieter, Dr. Dipl.-Chem.,**
**Nachtigallenweg 1a, D-6233 Kelkheim (DE)**

(54) **Elektrophotographisches Aufzeichnungsmaterial.**

(57) Die Erfindung betrifft ein elektrophotographisches Aufzeichnungsmaterial aus einem elektrisch leitenden Schichtträger, gegebenenfalls einer isolierenden Zwischenschicht und einer photoleitfähigen Einfachschicht mit einem Pyrimido-[5',4':5,6]-pyrido-[1,2-a]-benz-imidazol-Derivat als Photoleiter, Bindemittel und Farbstoff oder Pigment als Sensibilisator, dadurch gekennzeichnet, daß als Photoleiter eine Verbindung der allgemeinen Formel I vorhanden ist,

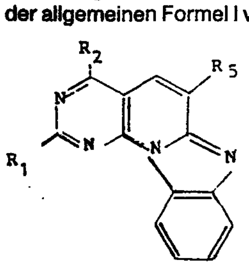

worin $R_1$ und $R_2$ gleich oder verschieden sind und Wasserstoff oder eine $R_3$, $R_4$N-Gruppe, $R_3$ und $R_4$ gleich oder verschieden sind und ($C_1$ bis $C_4$)-Alkyl, Hydroxy-($C_1$ bis $C_4$)-alkyl, gegebenenfalls ($C_1$ bis $C_4$)-alkylsubstituiertes Phenyl oder Benzyl oder zusammen mit dem N-Atom, an das sie gebunden sind, eine Imidazolyl- oder Triazolyl-Gruppe und $R_5$ eine Benzthiazol-, Benzoxazol- oder Benzimidazol-Gruppe bedeuten. Der Photoleiter ist überraschend im sichtbaren Bereich um 430 bis 500 nm photoempfindlich und kann mit Farbstoffen im längerwelligen Bereich sensibilisiert werden.

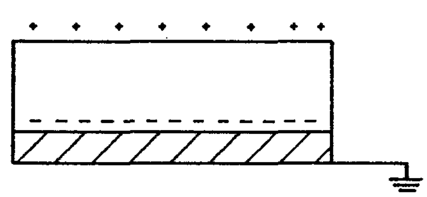

EP 0 191 318 A2

H O E C H S T   A K T I E N G E S E L L S C H A F T
KALLE Niederlassung der Hoechst AG

85/K 006

20. Januar 1986
WLK-Dr.S.-gv



ELEKTROPHOTOGRAPHISCHES AUFZEICHNUNGSMATERIAL

Die Erfindung betrifft ein elektrophotographisches Aufzeichnungsmaterial aus einem elektrisch leitenden Schichtträger, gegebenenfalls einer isolierenden Zwischenschicht und einer photoleitfähigen Einfachschicht aus Pyrimido-[5',4':5,6]-pyrido-[1,2-a]-benzimidazol-Derivaten als Photoleiter, Bindemittel und homogen gelöstem oder dispers verteiltem Farbstoff oder Pigment als Sensibilisator.

Die Erfindung betrifft insbesondere ein elektrophotographisches Aufzeichnungsmaterial für eine auf elektrophotographischem Wege herstellbare lithographische Druckform oder gedruckte Schaltung, wenn alkalisch entschichtbare Bindemittel in der photoleitfähigen Schicht vorhanden sind.

Photoleiterverbindungen der genannten Art sind bekannt. Sie wurden bereits in der DE-OS 29 29 414 als optische Aufheller, Fluoreszenzfarbstoffe oder auch als Photoleiter generell beschrieben.

Ihre außergewöhnlich guten photoleitfähigen Eigenschaften in vorwiegend alkalisch entschichtbaren Bindemitteln wurden dort jedoch weder beschrieben noch angedeutet. Der Anwendungsbereich der genannten Verbindungen war wegen ihrer geringen Löslichkeits- und Filmbildungseigenschaften sehr eingeschränkt.

Bei den meisten als organischen Photoleitern bekannten Verbindungen, wie zum Beispiel Oxazol-, Oxdiazol-, Pyrazolin-, Phenylhydrazon-Derivaten sowie Polyvinylcarbazol liegt die maximale Absorption unter einer Wellenlänge von $\lambda$ = 400 nm. Mit Bindemitteln sind die photoleitfähigen Schichten über $\lambda$ = 430 nm größtenteils transparent, so daß sie ohne Sensibilisierung nur im nahen UV-Bereich photoempfindlich sind.

Aufgabe der Erfindung war es, organische Verbindungen anzugeben, die zusammen mit geeigneten Bindemitteln, insbesondere alkalisch entschichtbaren, eine homogene Filmbildung gewährleisten und eine gute Photoempfindlichkeit aufweisen, die im sichtbaren Bereich von etwa 430 bis 500 nm liegt. Es war weiterhin erwünscht, daß diese organischen Verbindungen mit Sensibilisatoren und/oder Pigmenten spektral erweiterungsfähig sind.

Diese Aufgabe wird durch ein Aufzeichnungsmaterial der eingangs genannten Art gelöst, das dadurch gekennzeichnet ist, daß als Photoleiter eine Verbindung der im Formelanhang genannten allgemeinen Formel I, vorhanden ist, worin

$R_1$ und $R_2$ gleich oder verschieden sind und Wasserstoff oder eine $R_3R_4N$-Gruppe,

$R_3$ und $R_4$ gleich oder verschieden sind und $C_1$-$C_4$-Alkyl, Hydroxy-($C_1$-$C_4$)-alkyl, gegebenenfalls ($C_1$ bis $C_4$)alkylsubstituiertes Phenyl oder Benzyl oder zusammen mit dem

N-Atom, an das sie gebunden sind, eine Imidazolyl- oder Triazolyl-Gruppe, und

$R_5$ eine Benzthiazol-, Benzoxazol- oder Benzimidazol-Gruppe

bedeuten. Ganz besonders geeignet ist als Photoleiter eine Verbindung der Formel II (Formelanhang).

Im Gegensatz zu den bekannten Verbindungen, die durch Halogen oder Ethergruppen substituiert sind, lassen sich die erfindungsgemäßen Verbindungen sehr gut lösen und zu Filmen verarbeiten.

Sie weisen zusammen mit Filmbildnern bei positiver elektrostatischer Aufladung im Wellenlängenbereich bis 500 nm eine außergewöhnliche und überraschend hohe Eigenphotoempfindlichkeit auf, die sie besonders gut für bestimmte Laserlichtquellen (He/Cd- und Ar-Laser) geeignet macht.

Praktisch einsatzfähig sind sie mit Filmbildnern, wie Polyvinylacetalen, Polyesterharzen oder Vinylchlorid/Vinylacetat-Mischpolymerisaten für elektrophotographische Mikrofilmaufzeichnungsmaterialien. Mit alkalisch entschichtbaren Bindemitteln, wie Copolymerisaten aus Styrol und Maleinsäureanhydrid oder Vinylacetat, (Meth)acrylsäureestern und Crotonsäure oder Phenolharz allein oder in Mischung sind diese photoleitfähigen Verbindungen zu positiv aufladbaren, photoempfindlichen

HOECHST AKTIENGESELLSCHAFT
KALLE Niederlassung der Hoechst AG



Schichten für Druckformen oder gedruckte Schaltungen verarbeitbar.

Der spektrale Empfindlichkeitsbereich der erfindungsgemäßen elektrophotographischen Aufzeichnungsmaterialien läßt sich weiter je nach Anwendung durch homogen gelöste Sensibilisatoren oder durch photoaktive, dispers in der photoleitfähigen Schicht verteilte Pigmente beeinflussen.

Die Herstellung und Charakterisierung der erfindungsgemäßen photoleitfähigen Verbindungen geht aus DE-OS 29 29 414 hervor.

Man kann auch die Verbindung nach der allgemeinen Formel I durch Kondensation mit Formaldehyd zu einem Polykondensat umsetzen, das neben guten Filmbildungseigenschaften auch eine hohe Photoempfindlichkeit aufweist.

Mit einer Reihe von Bindemitteln lassen sich diese Verbindungen zusammen lösen und zu homogenen Filmen vergießen. Das Mischungsverhältnis Photoleiter/Bindemittel beträgt vorzugsweise 1:1. Es wird durch Auskristallisation bei einem zu hohen Anteil Photoleiter (größer als 50 %), bei einem zu niedrigen Anteil (kleiner als 25 %) durch die geringere Photoempfindlichkeit im jeweiligen Bindemittel begrenzt.

Die Photoleiterschichten nach der Erfindung können unsensibilisiert, wie in der beigefügten Figur 1 angezeigt, homogen sensibilisiert, bevorzugt mit homogen gelösten Sensibilisatoren oder Akzeptorzusätzen, etwa nach Figur 2, oder mit dispers verteilten Pigmentzusätzen als Ladungsträgererzeugungszentren vorliegen, wie dies durch Figur 3 dokumentiert wird.

Das hohe Absorptionsvermögen der erfindungsgemäßen photoleitfähigen Schichten im visuellen Wellenlängenbereich bis ca. 500 nm geht aus der beigefügten Figur 4 hervor (Kurve 2). Dabei liegt die maximale Absorption der Verbindung nach Formel II bei 431 nm mit einem Nebenmaximum bei 455 nm. Vergleichsweise wurde auch ein Film aus gleichem Bindemittel und 2,5-Bis(4-diethylaminophenyl)-oxdiazol-1,3,4 hergestellt, dessen Transmission ab ca. 425 nm stark ansteigt (Kurve 1).

Die hohe Photoempfindlichkeit bei positiver Aufladung kann durch Absorption, Anregung sowie Ladungserzeugung unter dem Einfluß des elektrischen Feldes in den Photoleitermolekülen der oberen Schichtzone erklärt werden, wobei die Anregungszone von der Eindringtiefe des Lichtes abhängt (Figur 7).

Die dabei erzeugten Defektelektronen werden aufgrund des p-leitenden Charakters (Ausbildung und Transport von Radikalkationen) dieser photoleitfähigen Verbindungen über den größten Dickenbereich besser transportiert, nach dem Schema:

$h\nu < 500$ nm

| | | |
|---|---|---|
| | $PL \rightarrow PL^*$ | Anregung |
| | $PL^* \rightarrow \cdot PL^{\oplus} + \ominus$ | (Ladungsträgererzeugung) |
| | $\cdot PL^{\oplus} + PL \rightarrow PL + \cdot PL^{\oplus}$ | (Ladungstransport). |

Als Schichtträger, insbesondere für die Herstellung von Druckformen auf elektrophotographischem Wege können sämtliche für diesen Zweck bekannten Materialien eingesetzt werden, wie zum Beispiel Aluminium-, Zink-, Magnesium-, Kupferfolien oder -platten oder Mehrmetallplatten. Es kommen auch Kunststoffe, wie zum Beispiel Polyamide in Folienform oder metallbedampfte Folien als Schichtträger in Frage. Besonders bewährt haben sich oberflächenveredelte Aluminiumfolien. Die Oberflächenveredelung besteht in einer mechanischen oder elektrochemischen Aufrauhung und gegebenenfalls in einer anschließenden Anodisierung und Behandlung mit Silikat oder mit Polyvinylphosphonsäure gemäß DE-OS 16 21 478 (entsprechend GB-PS 1,230,447).

Außerdem kommen aluminiumkaschierte Metall- oder aluminiumbedampfte Polyesterfolien für Kopiermaterialien, mit zum Beispiel Palladium- oder Indium-Zinn-Oxid leitfähig gemachte transparente Folien oder Spezialpapiere für elektrophotographische Mikrofilm-Aufzeichnungsmaterilien in Frage.

H O E C H S T   A K T I E N G E S E L L S C H A F T
KALLE Niederlassung der Hoechst AG



Die in elektrophotographischen Aufzeichnungsmaterialien gegebenenfalls enthaltene Zwischenschicht kann aus organischen Kunstharzen, wie Polyurethanen, in dünner Schichtdicke gebildet werden.

Als Bindemittel sind hinsichtlich der Filmeigenschaften und der Haftfestigkeit Natur- bzw. Kunstharze, insbesondere Polyesterharze, Polycarbonate, Polyurethane, Vinylchlorid/Vinylacetat-Copolymerisate, Polyvinylacetale, Polyvinylacetate, Celluloseacetobutyrate, Silikonharze, Poly(meth)acrylate, Cellulosenitrate, Kautschuk und Kautschukderivate, wie Chlorkautschuk und cyclisierter Kautschuk etc., geeignet. Bei ihrer Auswahl spielen außer den filmbildenden und elektrischen Eigenschaften sowie denen der Haftfestigkeit auf der Schichtträgerunterlage bei Einsatz für Druckformen oder gedruckte Schaltungen vor allem Löslichkeitseigenschaften eine besondere Rolle. Für praktische Zwecke sind solche Bindemittel besonders geeignet, die in wäßrigen oder alkoholischen Lösungsmittelsystemen, gegebenenfalls unter Säure- oder Alkalizusatz, löslich sind. Geeignete Bindemittel sind hiernach hochmolekulare Substanzen, die alkalilöslich machende Gruppen tragen. Solche Gruppen sind beispielsweise Säureanhydrid-, Carboxyl-, Phenol-, Sulfosäure-, Sulfonamid- oder Sulfonimidgruppen.

Mischpolymerisate mit Anhydridgruppen können mit besonders gutem Erfolg verwendet werden, da durch das Fehlen freier Säuregruppen die Dunkelleitfähigkeit der photo-

HOECHST AKTIENGESELLSCHAFT
KALLE Niederlassung der Hoechst AG



leitfähigen Schicht gering ist bei guter Alkalilöslichkeit. Ganz besonders geeignet sind Mischpolymerisate aus Styrol und Maleinsäureanhydrid sowie Phenolharze.

Als alkalilösliche Bindemittel können weiterhin Mischpolymerisate aus Styrol, Methacryläsure und Methacrylsäureester eingesetzt werden, mit einer Zusammensetzung von 1 bis 35 % Styrol, 10 bis 40 % Methacrylsäure und 35 bis 83 % Methacrylsäure-n-hexylester. Hervorragend geeignet ist ein Terpolymer aus 10 % Styrol, 30 % Methacrylsäure und 60 % Methacrylsäure-n-hexylester. Weiter sind Polyvinylacetate, insbesondere Copolymerisate aus Vinylacetat und Crotonsäure einsatzfähig.

Die Bindemittel können allein oder in Kombination zum Einsatz gelangen.

Die photoleitfähige Schicht enthält im wesentlichen die organische photoleitfähige Verbindung, Bindemittel, Sensibilisatoren und, fallweise, weitere übliche Zusätze, wie Aktivatoren, Weichmacher, Verlaufmittel und dergleichen.

Zur homogenen Sensibilisierung werden beispielsweise die in den Farbstofftabellen von Schultz (7. Auflage, 1. Band, 1931) aufgeführten organischen Farbstoffe eingesetzt, zum Beispiel Triarylmethanfarbstoffe, wie Brillantgrün (Nr. 760, S. 314), Victoriablau B (Nr. 822, S. 347), Methylviolett (Nr. 783, S. 327), Kristallviolett (Nr. 785, S. 329), Säureviolett 6B (Nr. 381, S. 351); Xanthenfarbstoffe, und zwar Rhodamine, wie Rhodamin B (Nr. 864, S. 365), Rhodamin 6G (Nr. 866, S. 366), Rhodamin G extra (Nr. 865, S. 366), Sulforhodamin B (Nr. 863, S. 364) und Echtsäureeosin G (Nr. 870, S. 368) sowie Phthaleine, wie Eosin S (Nr. 883, S. 375), Eosin A (Nr. 881, S. 374), Erythrosin (Nr. 866, S. 376), Phloxin (Nr. 890, S. 378), Rose bengale (Nr. 889, S. 378) und Fluorescein (Nr. 880, S. 373); Thiazinfarbstoffe, wie Methylenblau (Nr. 1038, S. 449); Acridinfarbstoffe, wie Acridingelb (Nr. 901, S. 383), Acridinorange (Nr. 908, S. 387) und Trypaflavin (Nr. 906, S. 386); Chinolinfarbstoffe, wie Pinacyanol (Nr. 924, S. 396) und Kryptocyanin (Nr. 927, S. 397); Chinonfarbstoffe und Ketonfarbstoffe, wie Alizarin (Nr. 1141, S. 449), Alizarinrot S (Nr. 1145, S. 502) und Chinizarin (Nr. 1148, S. 504); Cyaninfarbstoffe (Polymethinfarbstoffe), wie Astrazongelb 3G (Colour Index Nr. (C.I.) 48 055) und 5G (C.I. 48 065), Basic Yellow 52115 (C.I. 48 060), Astrazongelb GRL, Astrazonorange G (C.I. 48 040) und R (C.I. 48 035), Astrazonorange 3R (noch nicht klassifiziert).

Pyryliumsalze, Thiapyryliumsalze, Benzopyryliumsalze können ebenfalls verwendet werden.

Bevorzugt werden Farbstoffe eingesetzt, die im Bereich von 500 bis 750 nm absorbieren, aber auch Farbstoffe mit einem Absorptionsbereich von 400 bis 500 nm können empfindlichkeitssteigernd wirken.

Sensibilisierungsfarbstoffmischungen können ebenfalls vorliegen.

Als dispers verteilte Farbstoffe oder Pigmente können auch metallhaltige oder metallfreie Phthalocyaninpigmente, zum Beispiel Kupferphthalocyanin, Perinon-, Thioindigo-, höher annellierte Chinon-, Chinacridon-, Perylen-, Anthrachinon-, Dioxazin-, Azo-, Bisazo-, Trisazo-, Cyanin-Pigmente oder Benzo(thio)-xanthen-Derivate sowie deren Mischungen eingesetzt werden. Davon sind besonders bevorzugt Phthalocyaninpigmente, wie die verschiedenen Cu-Phthalocyaninmodifikationen (α, γ, ε), Bis- und Trisazopigmente, Perylimid-Pigmente sowie Kondensationsprodukte aus Perylen-3,4,9,10-tetracarbonsäureanhydrid und aromatischen Diaminen, wie o-Phenylendiamin, zum Beispiel Perylen-3,4,9,10-tetracarbonsäurediimidbisbenzimidazole, oder (Iso)Violanthrone. Sie können bis zu 30 %, bezogen auf die Gesamtmasse der photoleitfähigen Schicht, vorhanden sein, bevorzugt werden Mengen im Bereich von 0,1 bis 10 % Pigment verwendet.

Die Schichtdicke der Photoleiterschicht ist nicht kritisch und liegt im allgemeinen im Bereich von 2 bis 20 µm, kann aber je nach Anwendung diese Grenzen über- oder gegebenenfalls auch unterschreiten.

Die folgenden Beispiele sollen die Erfindung näher charakterisieren, ohne sie hierauf zu beschränken.

Beispiel 1

Eine 10 %ige Lösung aus 50 Teilen einer Verbindung gemäß Formel II und 50 Teilen Polyvinylbutyral (Mowital B30H) in Tetrahydrofuran (THF) wird auf eine aluminiumbedampfte Polyesterfolie in unterschiedlicher Schichtdicke geschleudert und anschließend während ca. 5 Minuten bei 90 bis 100 °C getrocknet.

Die Messung der Photoempfindlichkeit dieser homogenen photoleitfähigen Schichten wird folgendermaßen durchgeführt:

Zur Ermittlung der Hellentladungskurven bewegt sich die Meßprobe auf einem sich drehenden Teller durch eine Aufladevorrichtung hindurch zur Belichtungsstation, wo sie mit einer Xenonlampe XBO 150 kontinuierlich belichtet wird. Ein Wärmeabsorptionsglas und ein Neutralfilter sind der Lampe vorgeschaltet. Die Lichtintensität in der Meßebene liegt im Bereich von ca. 25 µW/cm$^2$. Die Aufladungshöhe und die photoinduzierte Hellabfallkurve werden über ein Elektrometer durch eine transparente Sonde oszillographisch aufgezeichnet. Die Photoleiterschicht wird durch die Aufladungshöhe ($U_o$) und diejenige Zeit ($T_{1/2}$) charakterisiert, nach der die Hälfte der Aufladung ($U_o/2$) erreicht ist. Das Produkt aus

HOECHST AKTIENGESELLSCHAFT
KALLE Niederlassung der Hoechst AG



$T_{1/2}$ und der gemessenen Lichtintensität I ($\mu W/cm^2$) ist die Halbwertsenergie $E_{1/2}$ ($\mu J/cm^2$):

| Schichtgewicht ($g/m^2$) | $U_o$(V) | $E_{1/2}$ |
|---|---|---|
| 4,7 (ca. 4 µm) | (-) 580 | 49 |
| | (+) 730 | 8,0 |
| 8,0 (ca. 7 µm) | (-) 780 | 65 |
| | (+) 710 | 11,5 |

Beispiel 2

In einer Lösung aus 47,5 Teilen einer Verbindung nach Formel II und 47,5 Teilen eines Mischpolymerisates aus Styrol und Maleinsäurenahydrid (Scripset 540) werden 5 Teile N,N'-Dimethylperylimid (C.I. Pigment Red 179) in einer Kugelmühle während ca. 5 Stunden sehr fein vermahlen. Die Pigmentdispersion wird anschließend auf drahtgebürstete Aluminiumfolie (100 µm) in 4 bis 5 µm Dicke geschichtet (Schicht Nr. 1). Desgleichen wird eine feindisperse Lösung aus gleichen Teilen obiger Verbindung sowie Mischpolymerisat (Scripset 540) und 2,5 Teilen metallfreiem Phthalocyanin (C.I. 74 100, Monolite Fast Blue GS) hergestellt und die Farbstoffdispersion sowohl auf drahtgebürstete (Schicht Nr. 2) als auch anodisierte Aluminiumfolie (Schicht Nr. 3), in etwa 5 µm Dicke nach Trocknung, geschleudert.

HOECHST AKTIENGESELLSCHAFT
KALLE Niederlassung der Hoechst AG



Die Photoempfindlichkeit, analog Beispiel 1 vermessen, ist aus der folgenden Tabelle ersichtlich:

| Schicht Nr. | (+) $U_o$/V | $E_{1/2}$ | (-) $U_o$(V) | $E_{1/2}$ |
|---|---|---|---|---|
| 1 | 310 | 6,8 | 420 | 9,6 |
| 2 | 340 | 10,9 | - | - |
| 3 | 530 | 12,1 | - | - |

Beispiel 3

Zu einer Lösung aus gleichen Gewichtsteilen der Verbindung nach Formel II und Polyvinylbutyral (Mowital B30H) in THF werden lösliche Sensibilisierungsfarbstoffe, zum Beispiel Rhodamin B und Kristallviolett, im Gewichtsverhältnis 1:500, bezogen auf die photoleitfähige Verbindung, gegeben und die homogenen Farbstofflösungen auf Aluminiumfolie in 7 bis 8 µm Dicke geschleudert.

Die Messung der Photoempfindlichkeit dieser sensibilisierten Schichten ergibt folgende Werte:

H O E C H S T   A K T I E N G E S E L L S C H A F T
KALLE Niederlassung der Hoechst AG



| | Schicht (Sensib.) | (+) $U_o$(V) | $E_{1/2}$ | (-) $U_o$/V | $E_{1/2}$ |
|---|---|---|---|---|---|
| 1 | - | 780 | 11,0 | | |
| 2 | Rhodamin B | 820 | 9,2 | | |
| 3 | Kristall-violett | 820 | 7,3 | 710 | 19,1 |

Die spektrale Photoempfindlichkeit der Schichten 1 und 3 wurde unter Vorschaltung von Interferenzfiltern nach der in Beispiel 1 angegebenen Methode bestimmt.

Bei positiver Aufladung (800 bis 850 V) wird durch Belichten die Halbwertzeit ($T_{1/2}$ in msec) für den jeweiligen Wellenlängenbereich bestimmt. Durch Auftragen der reziproken Halbwertsenergie ($1/E_{1/2}$) gegen die Wellenlänge $\lambda$ in nm erhält man die spektrale Photoempfindlichkeitskurve. Dabei bedeutet die Halbwertsenergie $E_{1/2}$ diejenige Lichtenergie, die eingestrahlt werden muß, um die Schicht auf die Hälfte der Anfangsspannung $U_o$ zu entladen.

In Figur 5 sind die spektralen Photoempfindlichkeiten von Photoleiterschichten ohne (Kurve 1) und mit einem Sensibilisator (Kristallviolett, Kurve 2) bei positiver Aufladung aufgezeichnet.

HOECHST AKTIENGESELLSCHAFT
KALLE Niederlassung der Hoechst AG



Beispiel 4

Eine Lösung aus 65 Teilen einer Verbindung gemäß Formel II und 35 Teilen von Cellulosenitrat H4 in THF wird mit verschiedenen Sensibilisatoren, wie Kristallviolett, Astrazonorange R, Brillantgrün, im Verhältnis 1:50, bezogen auf obige Verbindung, versetzt und in 6 bis 7 µm Dicke (trocken) auf drahtgebürstete Aluminiumfolie geschleudert.

Nach dem Trocknen der Schichten wurden folgende Photoempfindlichkeiten, gemessen entsprechend Beispiel 1, festgestellt:

| Schicht mit Sensibilisator | (+) $U_o$(V) | $E_{1/2}$ |
|---|---|---|
| - | 670 | 12,9 |
| Kristallviolett | 690 | 2,5 |
| Astrazonorange R | 710 | 3,3 |
| Brillantgrün | 650 | 6,2 |

Beispiel 5

Zu einer Beschichtungslösung, wie in Beispiel 4 beschrieben, wird ein Sensibilisator Rhodamin B in unterschiedlichen Mengen (bezogen auf die Verbindung nach Formel II) zugegeben und daraus homogene Photoleiterschichten mit einer Dicke von 6 bis 7 µm hergestellt.

Die Messung der Photoempfindlichkeit erfolgt gemäß Beispiel 1:

| Schicht mit Rhodamin B Verhältnis | (+) $U_o(V)$ | $E_{1/2}$ | (-) $U_o/V$ | $E_{1/2}$ |
|---|---|---|---|---|
| - | 630 | 11,8 | 650 | 30 |
| 1:1000 | 610 | 5,4 | 500 | 18,9 |
| 1: 500 | 680 | 3,8 | 710 | 11,2 |
| 1: 100 | 670 | 2,4 | 640 | 7,1 |
| 1: 50+ | 640 | 2,1 | 680 | 7,2 |
| 1: 10 | 660 | 2,6 | 630 | 25,0 |

+ Von der Photoleiterschicht mit dem Sensibilisierungsverhältnis 1:50 wird die spektrale Photoempfindlichkeit bei positiver Aufladung (600V) wie in Beispiel 3 bestimmt (Figur 5, Kurve 3).

Beispiel 6

Eine Lösung aus gleichen Gewichtsteilen der Verbindung gemäß Formel II und Styrol/Maleinsäureanhydrid-Mischpolymerisat (Scripset 540) in THF wird mit unterschiedlichen Sensibilisatormengen Rhodamin B versetzt. Danach wird die homogene Lösung auf drahtgebürstete Aluminiumfolie in ca. 5 µm Dicke geschleudert und die Photoempfindlichkeit der Schichten nach Beispiel 1 bestimmt:

| Schicht mit Rhodamin B Verhältnis | (+) $U_o$(V) | $E_{1/2}$ |
|---|---|---|
| -+ | 710 | 7,3 |
| 1:500 | 710 | 6,6 |
| 1:100 | 680 | 5,1 |
| 1: 50+ | 770 | 4,5 |

+ Von der nicht sensibilisierten sowie 1:50 sensibilisierten Photoleiterschicht wurden die spektralen Photoempfindlichkeitskurven bei positiver Aufladung (500 V) ermittelt (Figur 6, Kurven 1 und 2).

Vergleichsbeispiel

In der gleichen Zusammensetzung, wie in Beispiel 6 beschrieben, und unter Sensibilisierungsbedingungen (1:500) wurden die bekannten photoleitfähigen Verbindungen 2,5-Bis(4'-diethylaminophenyl)-oxdiazol-1,3,4 (A), 2-Phenyl-4-(2'-chlorphenyl)-5-(4'-diethylaminophenyl)-oxazol (B) sowie 2-Vinyl-4-(2'-chlorphenyl)-5-(4'-diethylaminophenyl)-oxazol (C) in ca. 5 µm Dicke auf das gleiche Trägermaterial geschleudert. Die Vermessung der Photoempfindlichkeit ergab folgende Werte:

HOECHST AKTIENGESELLSCHAFT
KALLE Niederlassung der Hoechst AG



| Photoleiterschicht (Rhodamin B) | (+) $U_o$(V) | $E_{1/2}$ |
|---|---|---|
| A | 650 | 25,9 |
| + (1:500) | 700 | 12,7 |
| B | 590 | 17,9 |
| + (1:500) | 670 | 9,0 |
| C | 640 | 35,6 |
| + (1:500) | 830 | 12,5 |

Beispiel 7

Verbindung gemäß Formel II (0,01 Mol) wurde mit Paraformaldehyd (0,01 Mol) in Eisessig unter Zusatz von 0,5 g wasserfreiem $ZnCl_2$ schonend (weniger als 40 °C) während ca. 2 Stunden umgesetzt. Nach Abtrennung der anorganischen Bestandteile sowie der unlöslichen und monomeren organischen Anteile wurde mit dem löslichen Anteil (weniger als 20 %) ein homogener Film in ca. 2 µm Dicke auf drahtgebürstete Aluminiumfolie gegossen.

Die Messung der Photoempfindlichkeit bei positiver Aufladung von 190 V ergab einen $E_{1/2}$-Wert von 4,65 µJ/cm$^2$.

-------

HOECHST AKTIENGESELLSCHAFT
KALLE Niederlassung der Hoechst AG



PATENTANSPRÜCHE

1. Elektrophotographisches Aufzeichnungsmaterial aus einem elektrisch leitenden Schichtträger, gegebenenfalls einer isolierenden Zwischenschicht und einer photoleitfähigen Einfachschicht mit einem Pyrimido-[5',4':5,6]-pyrido-[1,2-a]-benz- imidazol-Derivat als Photoleiter, Bindemittel und Farbstoff oder Pigment als Sensibilisator, dadurch gekennzeichnet, daß als Photoleiter eine Verbindung der allgemeinen Formel I (Formelanhang) vorhanden ist,

worin $R_1$ und $R_2$ gleich oder verschieden sind und Wasserstoff oder eine $R_3,R_4N$-Gruppe,

$R_3$ und $R_4$ gleich oder verschieden sind und ($C_1$ bis $C_4$)-Alkyl, Hydroxy-($C_1$ bis $C_4$)-alkyl, gegebenenfalls ($C_1$ bis $C_4$)-alkylsubstituiertes Phenyl oder Benzyl oder zusammen mit dem N-Atom, an das sie gebunden sind, eine Imidazolyl- oder Triazolyl-Gruppe

und

$R_5$ eine Benzthiazol-, Benzoxazol- oder Benzimidazol-Gruppe

bedeuten.

2. Aufzeichnungsmaterial nach Anspruch 1, dadurch gekennzeichnet, daß als Photoleiter eine Verbindung der Formel II (Formelanhang), worin $R_1$ und $R_2$ mit $R_3$ und $R_4$ je einen Diethylaminorest und $R_5$ eine Benzthiazolgruppe bedeuten, vorhanden ist.

H O E C H S T   A K T I E N G E S E L L S C H A F T
KALLE Niederlassung der Hoechst AG



3. Aufzeichnungsmaterial nach Ansprüchen 1 oder 2, dadurch gekennzeichnet, daß als Bindemittel Copolymerisate aus Styrol und Maleinsäureanhydrid, aus Vinylacetat und Crotonsäure oder Phenolharz allein oder in Mischung vorhanden sind.

4. Aufzeichnungsmaterial nach Ansprüchen 1 oder 2, dadurch gekennzeichnet, daß als Sensibilisator in der photoleitfähigen Schicht homogen gelöster Farbstoff vorhanden ist.

5. Aufzeichnungsmaterial nach Ansprüchen 1 oder 2, dadurch gekennzeichnet, daß als Sensibilisator in der photoleitfähigen Schicht dispers verteiltes Pigment vorhanden ist.

-------

H O E C H S T   A K T I E N G E S E L L S C H A F T
KALLE Niederlassung der Hoechst AG



FORMELANHANG

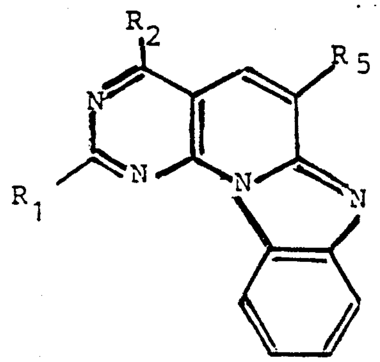

I

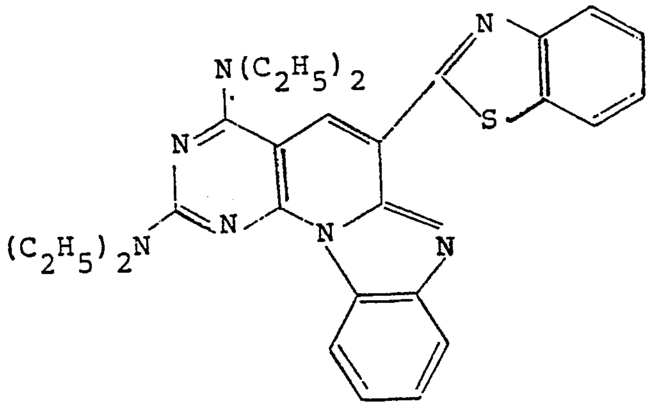

II

FIG. 1

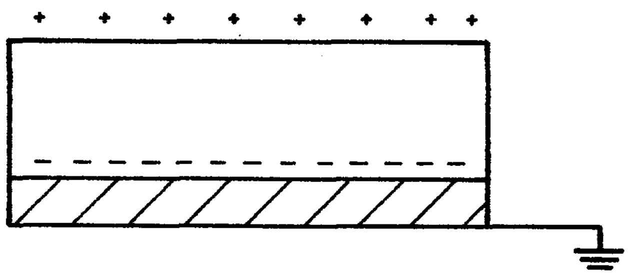

FIG. 2

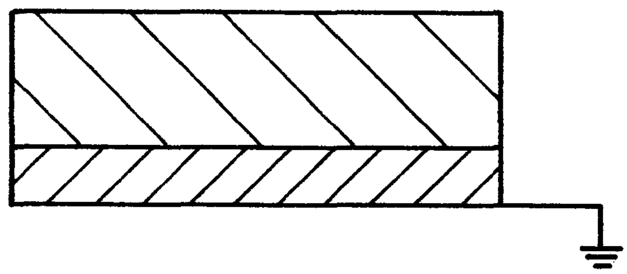

FIG. 3

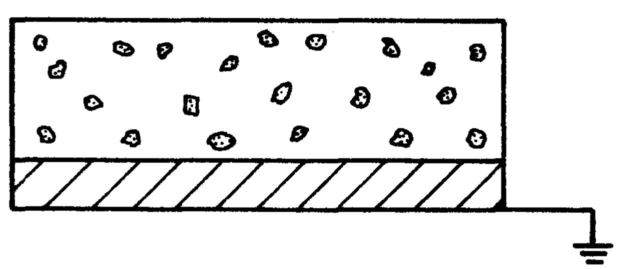

HOECHST AKTIENGESELLSCHAFT 85/K 006

FIG. 4

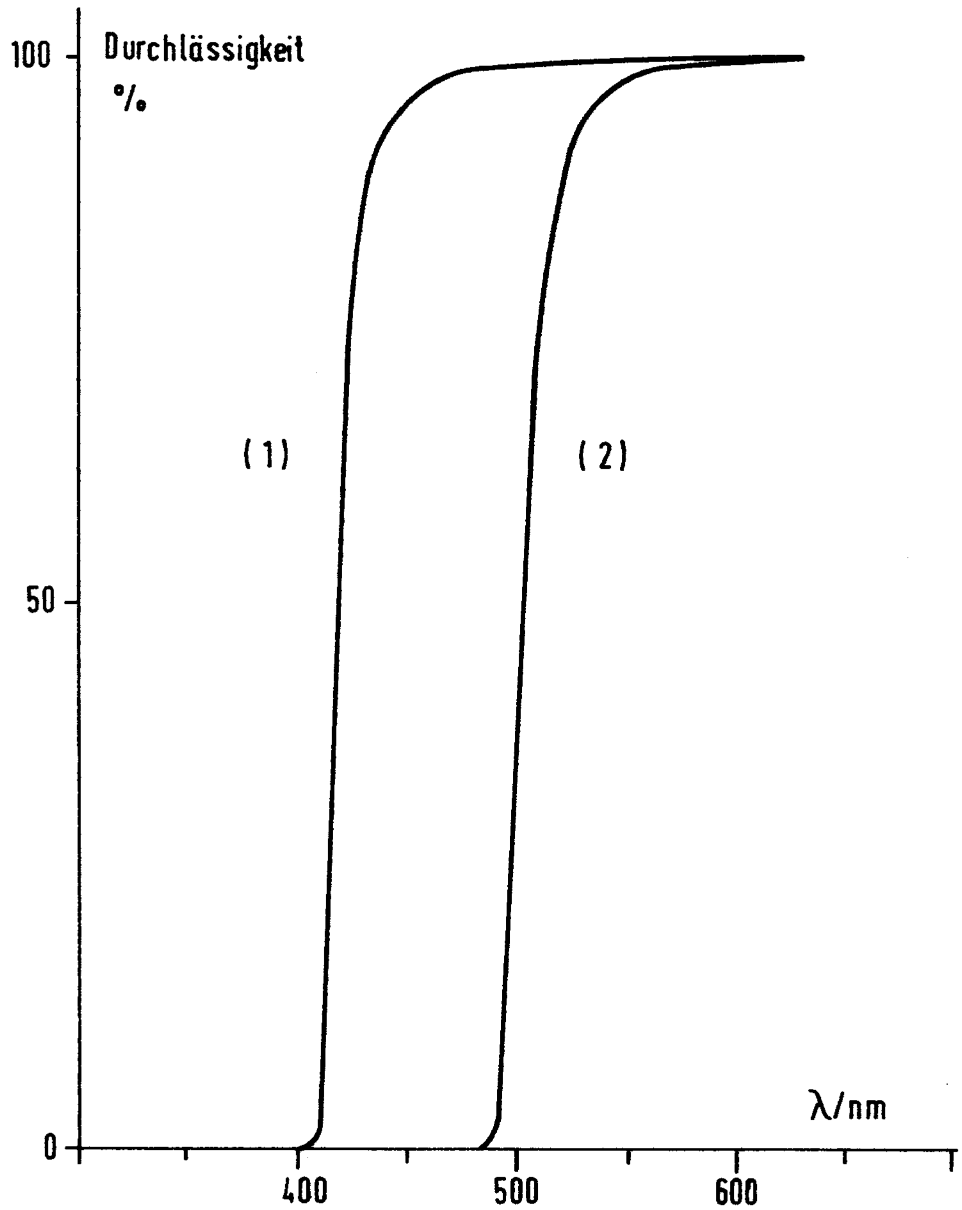


HOECHST AKTIENGESELLSCHAFT 85/K 006

FIG. 5

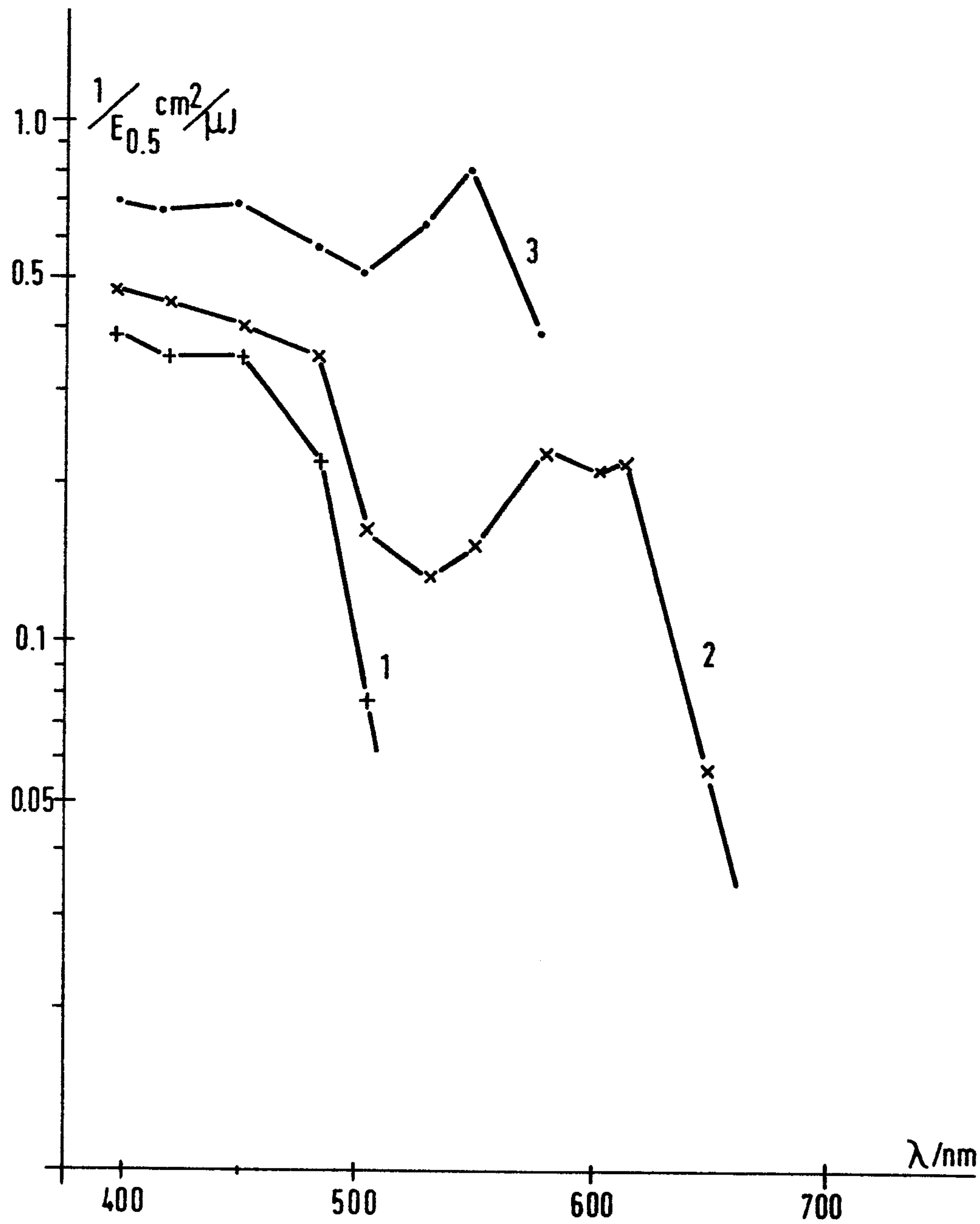

FIG. 6

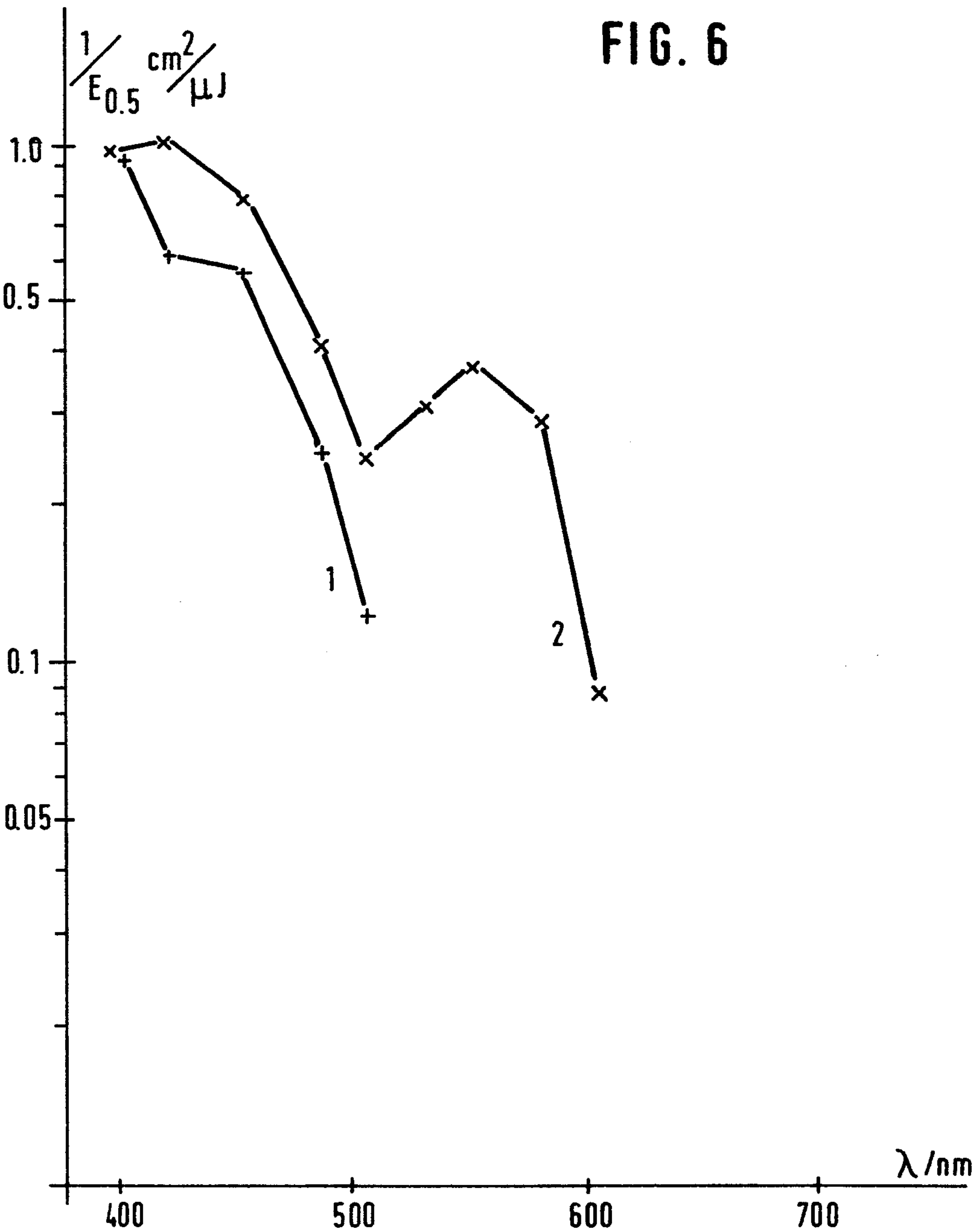

FIG. 7

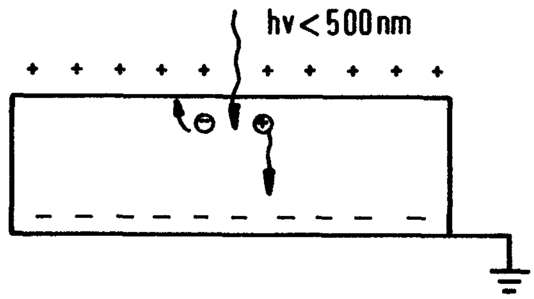


HOECHST AKTIENGESELLSCHAFT 85/K 006

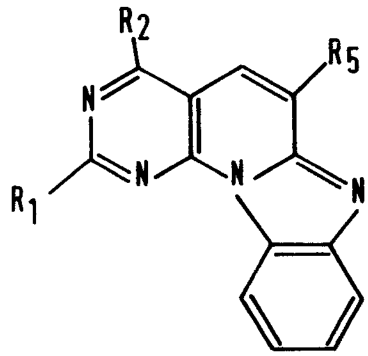

I

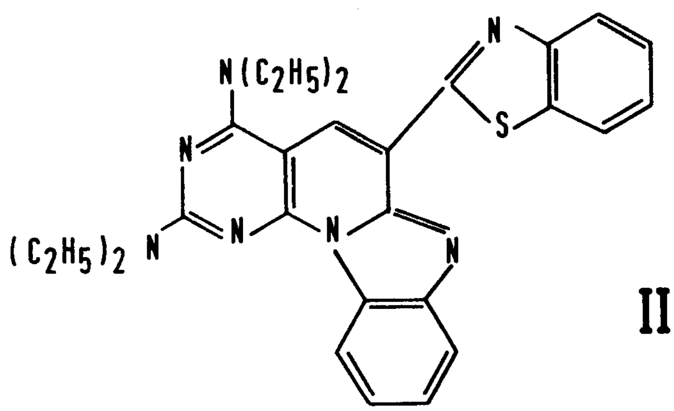

II

HOECHST AKTIENGESELLSCHAFT 85/K 006